(19) Europäisches Patentamt
European Patent Office
Office européen des brevets

(11) Numéro de publication : **0 138 721**
**B1**

(12) # FASCICULE DE BREVET EUROPÉEN

(45) Date de publication du fascicule du brevet : **26.04.89**

(51) Int. Cl.[4] : **C 07 D207/48**, C 07 D211/96, C 07 D223/10, A 61 K 31/395

(21) Numéro de dépôt : **84402099.0**

(22) Date de dépôt : **18.10.84**

(54) **Nouveaux benzenesulfonyl-lactames, leur procédé de préparation et leur application comme substance active de compositions pharmaceutiques.**

(30) Priorité : **18.10.83 FR 8316554**

(43) Date de publication de la demande : **24.04.85 Bulletin 85/17**

(45) Mention de la délivrance du brevet : **26.04.89 Bulletin 89/17**

(84) Etats contractants désignés : **BE CH DE FR GB IT LI LU NL SE**

(56) Documents cités :
**EP-A- 0 008 367**
**EP-A- 0 064 445**
**US-A- 3 453 289**
**CHEMICAL ABSTRACTS, vol. 94, no. 19, 11 mars 1981, page 642, no. 156735v, Columbus, Ohio, US; & JP - A - 80 81857 (KYOWA HAKKO KOGYO CO., LTD.) 20-06-1980**
**CHEMICAL ABSTRACTS, vol. 94, no. 24, 15 juin 1981, page 585, no. 208700b, Columbus, Ohio, US; & JP - A - 80 153 763 (KYOWA HAKKO KOGYO CO., LTD.) 29-11-1980**

(73) Titulaire : **DROPIC Société Civile de Gestion de Droit de Propriété Industrielle CHOAY**
**10 Avenue Matignon**
**F-75008 Paris (FR)**

(72) Inventeur : **Roger, Pierre**
**6, rue Paul Valéry**
**F-78423 Montigny les Bretonneux (FR)**
Inventeur : **Choay, Patrick**
**7 Cour Jasmin**
**F-75106 Paris (FR)**
Inventeur : **Fournier, Jean-Paul**
**10, rue Fontenay**
**F-78000 Versailles (FR)**

(74) Mandataire : **Gutmann, Ernest et al**
**S.C. Ernest Gutmann - Yves Plasseraud 67, boulevard Haussmann**
**F-75008 Paris (FR)**

**EP 0 138 721 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

L'invention est relative à de nouveaux médicaments qui présentent une activité sur le système nerveux central, notamment psychotrope avec modification du comportement et qui contiennent, à titre de principe actif, des composés chimiques dont la structure de base est du type benzènesulfonyl-lactame, ainsi que leurs sels obtenus avec des acides physiologiquement acceptables.

On appellera ci-après « médicament » toute composition pharmaceutique contenant en association avec un véhicule pharmaceutiquement acceptable l'un au moins des composés chimiques tels que définis ci-après.

L'invention est également relative aux composés nouveaux qui constituent la substance active des nouveaux médicaments.

L'invention est aussi relative à un procédé de préparation des susdits composés chimiques.

Des benzènesulfonyl-lactames de formule :

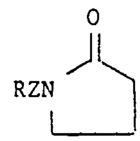

dans laquelle R représente $(R^2)_nC_6H_5\ (CH_2)_m$-, $R^2$ représente H, un radical alcoyle, alcoxy, un atome d'halogène ou un groupe nitro, n varie de 1 à 5, m prend les valeurs 0 ou 1, Z peut représenter $SO_2$, sont mentionnés dans les Chemical Abstracts, vol. 94, n° 156735v, 1981, p. 642.

Ces composés sont présentés comme étant des agents conservateurs de fruits.

Les médicaments selon l'invention sont caractérisés en ce qu'ils contiennent comme substance active l'un au moins des composés répondant à la formule générale (I) suivante :

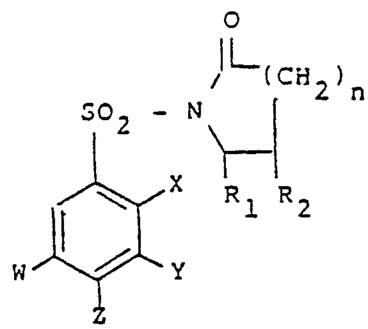

(I)

dans laquelle :

—X représente un atome d'hydrogène, un groupe alcoxy de 1 à 4 atomes de carbone ;

—Y représente un atome d'hydrogène, un groupe $CF_3$, $NO_2$ ;

—Z représente un atome d'hydrogène, un atome d'halogène, un groupe alcoxy de 1 à 4 atomes de carbone, un groupe $NO_2$ ;

—W représente un atome d'hydrogène, un atome d'halogène, un groupe $CF_3$, $NO_2$, un groupe alcoxy de 1 à 4 atomes de carbone ;

—n vaut 1, 2 ou 3 ;

—$R_1$ représente un atome d'hydrogène, un groupe alcoyle de 1 à 6 atomes de carbone ;

—$R_2$ représente un atome d'hydrogène, le groupe OH, un groupe OR

dans lequel R représente un groupe alcoyle de 1 à 4 atomes de carbone ou un groupe acyle de 1 à 6 atomes de carbone ;

sous réserve que X ou Z représente un groupe alcoxy de 1 à 4 atomes de carbone ou que Y ou W représente le groupe $CF_3$ ou $NO_2$.

Ce groupe sera dans la suite désigné par G1. Un groupe préféré de médicaments selon l'invention est constitué par ceux dont la substance active est un composé de formule générale (I) :

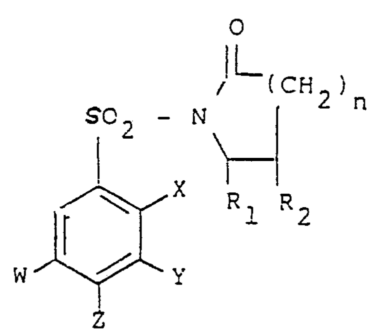

dans laquelle :

—X représente un atome d'hydrogène, un groupe alcoxy de 1 à 4 atomes de carbone ;

—Y représente un atome d'hydrogène, un groupe $CF_3$, $NO_2$ ;

—Z représente un atome d'hydrogène, un atome d'halogène, un groupe alcoxy de 1 à 4 atomes de carbone, un groupe $NO_2$ ;

—W représente un atome d'hydrogène, un atome d'halogène, un groupe $CF_3$, $NO_2$, un groupe alcoxy de 1 à 4 atomes de carbone ;

—n vaut 1, 2 ou 3 ;

—$R_1$ représente un atome d'hydrogène, un groupe alcoyle de 1 à 6 atomes de carbone ;

—$R_2$ représente un atome d'hydrogène, le groupe OH, un groupe OR dans lequel R représente un groupe alcoyle de 1 à 4 atomes de carbone ou un groupe acyle de 1 à 6 atomes de carbone ;

sous réserve que :

—lorsque n vaut 1, Y ou W représente le groupe $CF_3$ et

—lorsque n vaut 2 ou 3, X ou Z représente un groupe alcoxy de 1 à 4 atomes de carbone ou bien Y ou W représente le groupe $CF_3$ ou $NO_2$.

Ce groupe sera dans la suite désigné par G1bis.

Un groupe préféré de médicaments selon l'invention est constitué par ceux dont la substance active est un composé de formule générale (I), dans laquelle n vaut 1 et répondant à la formule générale suivante (II) :

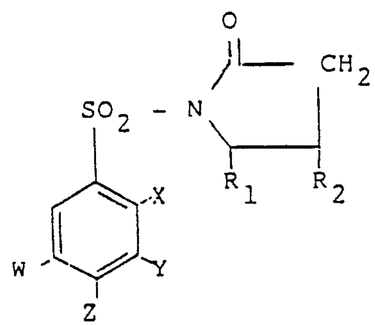

(II)

Ce groupe sera dans la suite désigné par G2.

Un autre groupe préféré de médicaments selon l'invention est constitué par ceux dont la substance active est un composé de formule générale (I) dans laquelle n vaut 2 et répondant à la formule générale suivante (III) :

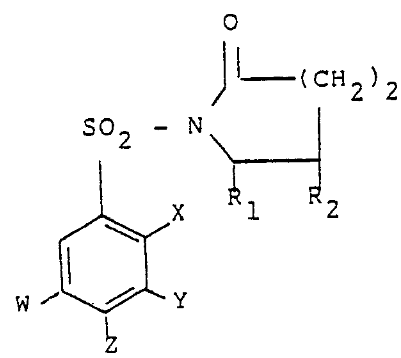

(III)

Ce groupe sera dans la suite désigné par G3.

Un autre groupe de médicaments préférés selon l'invention est constitué par ceux dont la substance active est un composé de formule générale (I), dans laquelle n vaut 3 et répondant à la formule générale suivante (IV) :

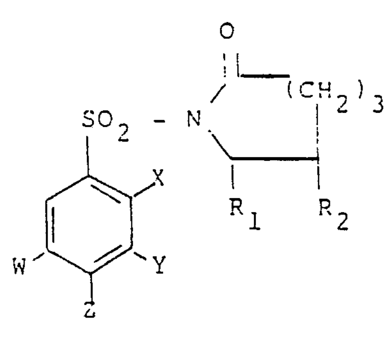

(IV)

Ce groupe sera dans la suite désigné par G4.

Un autre groupe préféré de médicaments selon l'invention est constitué par ceux dont la substance active est un composé de formule générale (I), dans laquelle $R_1$ représente H ou $CH_3$.

Ce groupe sera dans la suite désigné par G5.

Un autre groupe de médicaments préférés selon l'invention est constitué par ceux dont la substance active est un composé de formule générale (I), dans laquelle $R_2$ représente H, OH ou $OCOCH_3$.

Ce groupe sera dans la suite désigné par G6.

Un autre groupe de médicaments préférés selon l'invention est constitué par ceux dont la substance active est un composé de formule générale (I), dans laquelle Y représente $CF_3$, X représente H, et répondant à la formule générale suivante (VII) :

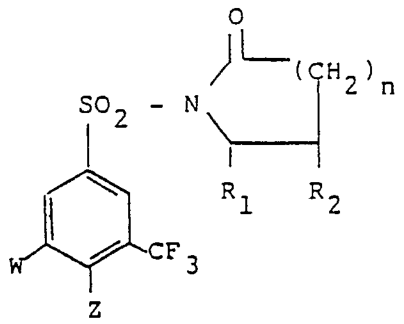

(VII)

et dans laquelle Z représente H ou C1 et W représente H ou $CF_3$.

Ce groupe sera dans la suite désigné par G7.

Parmi le groupe G7, un groupe préféré de médicaments selon l'invention est constitué par ceux dont la substance active est un composé dans lequel Z représente H et W représente $CF_3$ et répondant à la formule générale (VIII) suivante :

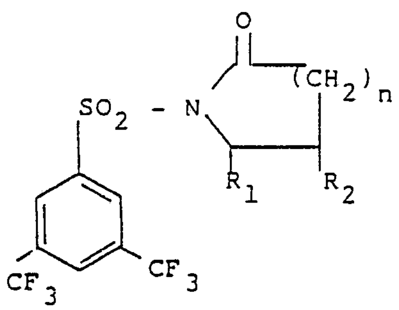

(VIII)

Ce groupe sera dans la suite désigné par G8.

Parmi le groupe G7, un autre groupe préféré de médicaments selon l'invention est constitué par ceux dont la substance active est un composé dans lequel Z représente Cl et W représente H et répondant à la formule générale (IX) suivante :

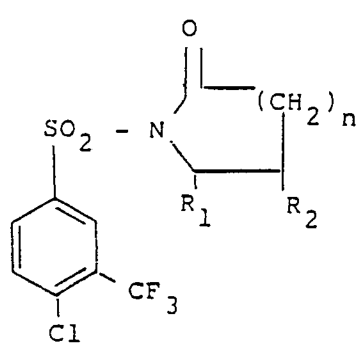

(IX)

Ce groupe sera dans la suite désigné par G9.

Un autre groupe de médicaments préférés selon l'invention est constitué par ceux dont la substance active est un composé de formule générale (I), dans laquelle X représente $OCH_3$, Y représente H et répondant à la formule générale (X) suivante :

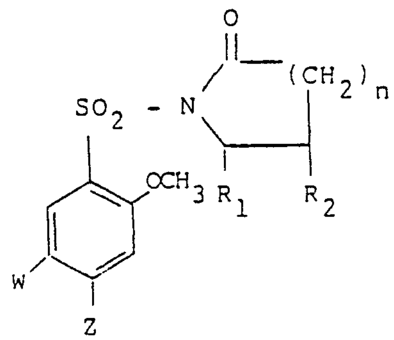

(X)

et dans laquelle Z représente H ou $OCH_3$ et W représente H, Cl ou $OCH_3$.

Ce groupe sera dans la suite désigné par G10.

Parmi le groupe G10, un groupe de médicaments préférés selon l'invention est constitué par ceux dont la substance active est un composé dans lequel Z représente H et W représente Cl et répondant à la formule générale (XI) suivante :

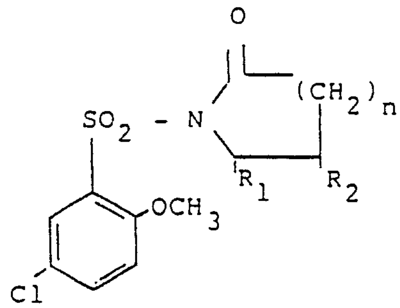

(XI)

Ce groupe sera dans la suite désigné par G11.

Parmi le groupe G10, un groupe de médicaments préférés selon l'invention est constitué par ceux dont la substance active est un composé dans lequel Z représente $OCH_3$ et W représente H et répondant à la formule générale (XII) suivante :

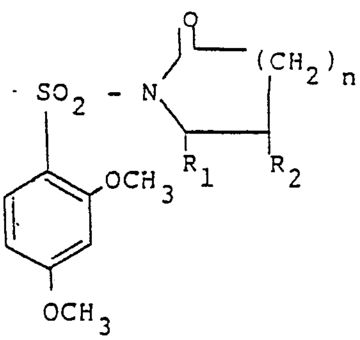

(XII)

Ce groupe sera dans la suite désigné par G12.

Parmi le groupe G10, un autre groupe préféré de médicaments selon l'invention est constitué par ceux dont la substance active est un composé dans lequel Z représente $OCH_3$ et W représente $OCH_3$ et répondant à la formule générale (XIII) suivante :

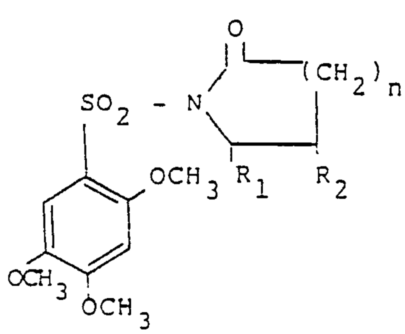

(XIII)

Ce groupe sera dans la suite désigné par G13.

Parmi le groupe G10, un autre groupe de médicaments préférés selon l'invention est constitué par ceux dont la substance active est un composé dans lequel Z représente $OCH_3$ et W représente C1 et répondant à la formule générale (XIV) suivante :

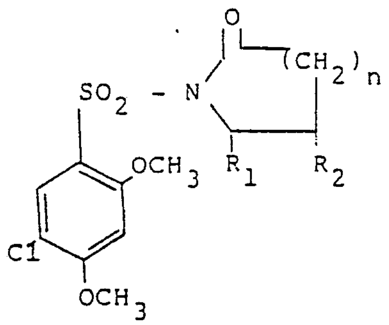

(XIV)

Ce groupe sera dans la suite désigné par G14.

Un autre groupe préféré de médicaments selon l'invention est constitué par ceux dont la substance active est un composé de formule générale (I), dans laquelle X représente H, Y représente H, Z représente $OCH_3$ et W représente H, et répondant à la formule générale (XV) suivante :

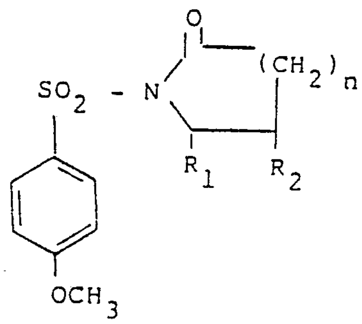

(XV)

Ce groupe sera dans la suite désigné par G15.

Un autre groupe de médicaments préférés selon l'invention est constitué par ceux dont la substance active est un composé de formule générale (I), dans laquelle Y représente $NO_2$ et répondant à la formule générale (XVI) suivante :

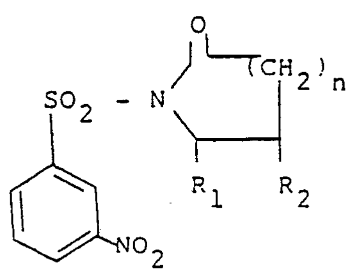

(XVI)

Ce groupe sera dans la suite désigné par G16.

Un groupe préféré de médicaments selon l'invention est constitué par ceux de formule :

(Voir Formules pages 7 et 8)

Composés n°

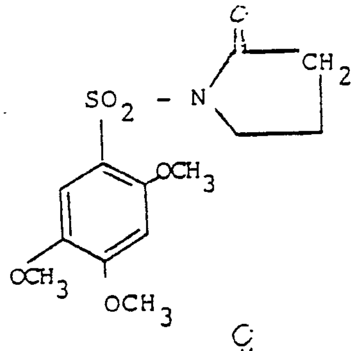

1 309

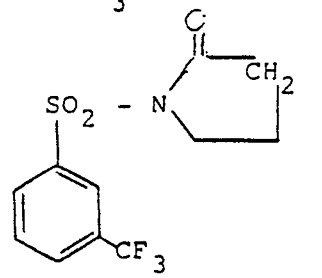

1 043

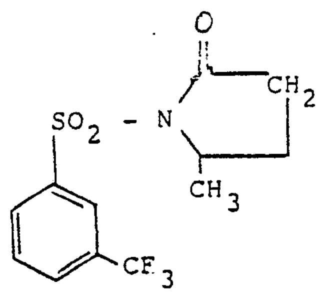

1 276

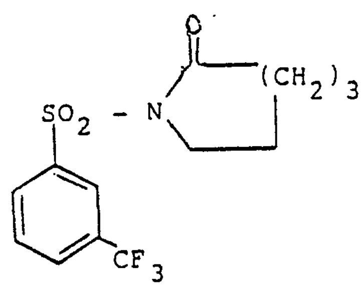

1 277

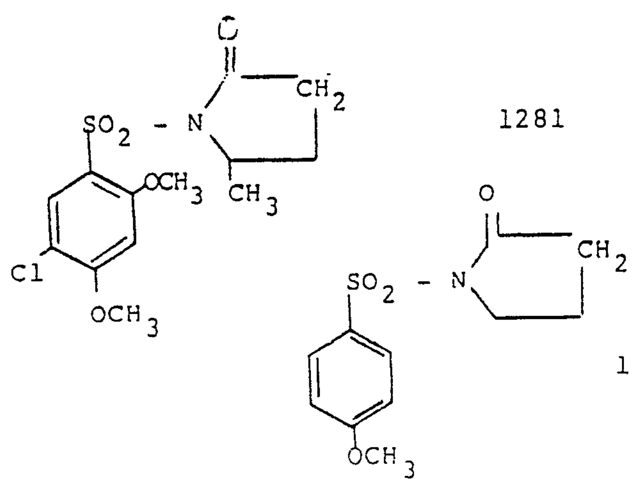

1281

1 120

Composés n°

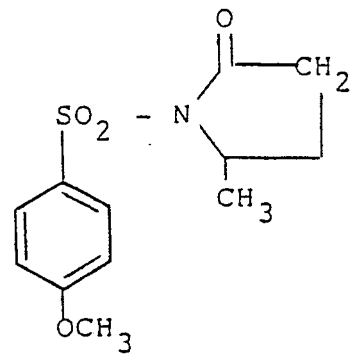

1 288

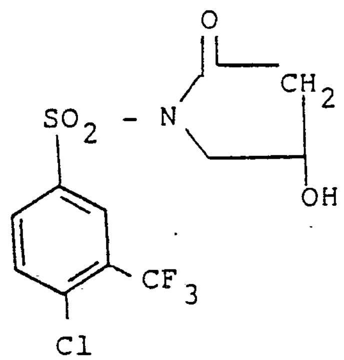

1 417

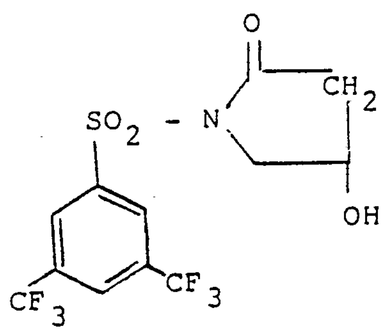

1 418

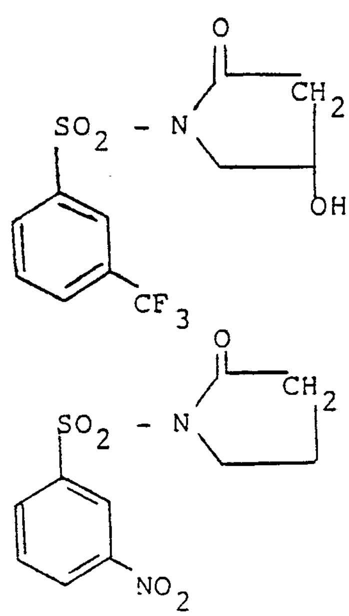

1 419

1 039

Les composés entrant dans les médicaments selon l'invention et qui sont nouveaux répondent à la formule générale (I) :

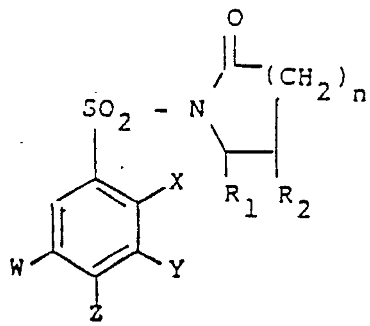

(I)

dans laquelle :

— X représente un atome d'hydrogène, un groupe alcoxy de 1 à 4 atomes de carbone ;

— Y représente un atome d'hydrogène, un groupe $CF_3$, $NO_2$ ;

— Z représente un atome d'hydrogène, un atome d'halogène, un groupe alcoxy de 1 à 4 atomes de carbone, un groupe $NO_2$ ;

— W représente un atome d'hydrogène, un atome d'halogène, un groupe $CF_3$, $NO_2$, un groupe alcoxy de 1 à 4 atomes de carbone ;

— n vaut 1, 2 ou 3 ;

— $R_1$ représente un atome d'hydrogène, un groupe alcoyle de 1 à 6 atomes de carbone ;

— $R_2$ représente un atome d'hydrogène, le groupe OH, un groupe OR dans lequel R représente un groupe alcoyle de 1 à 4 atomes de carbone ou un groupe acyle de 1 à 6 atomes de carbone ;

sous réserve que :

— lorsque n vaut 1, Y ou W représente le groupe $CF_3$ ; et

— lorsque n vaut 2 ou 3, X ou Z représente un groupe alcoxy de 1 à 4 atomes de carbone ou bien Y ou W représente le groupe $CF_3$ ou $NO_2$.

Les produits industriels nouveaux selon l'invention qui viennent d'être définis, correspondent au groupe G1 bis de médicaments ci-dessus définis.

Des classes préférées de composés selon l'invention sont constituées par les composés appartenant aux groupes G1 à G9 définis ci-dessus, à propos des médicaments, et dans lesquels lorsque n vaut 1, Y ou W représente $CF_3$ et lorsque n vaut 2 ou 3, ou bien X ou Z représente $OR_1$ ou bien Y ou W représente $CF_3$ ou $NO_2$.

Une autre classe préférée de composés selon l'invention est constituée par les composés de formule générale (X) ci-dessus indiquée, dans laquelle n vaut 2 ou 3, Z représente H ou $OCH_3$, et W représente H, Cl ou $OCH_3$.

Une autre classe préférée de composés selon l'invention est constituée par les composés de formules générales (XI), (XII), (XIII), (XIV), (XV) ou (XVI) ci-dessus représentées et dans lesquelles n vaut 2 ou 3.

Un groupe de composés préférés est constitué par ceux de formule :

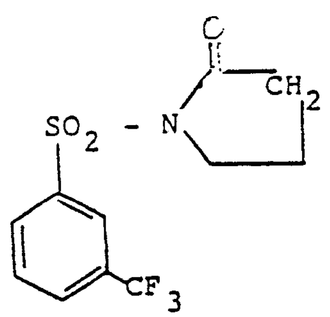

Composés n°

1 043

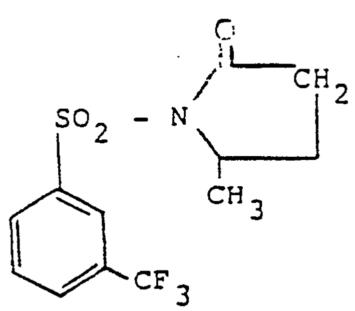

1 276

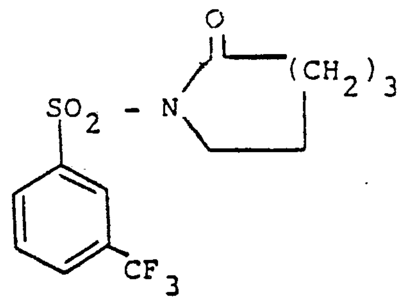

1 277

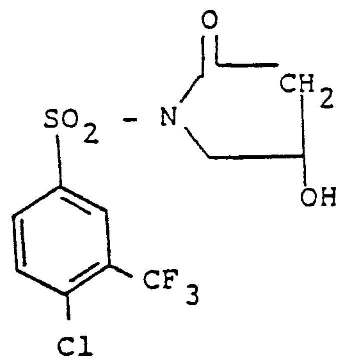

1 417

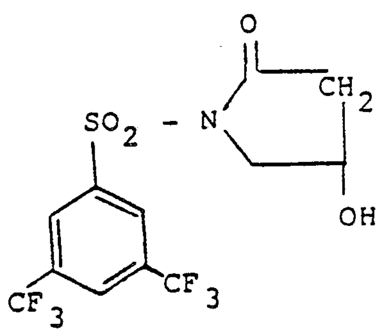

1 418

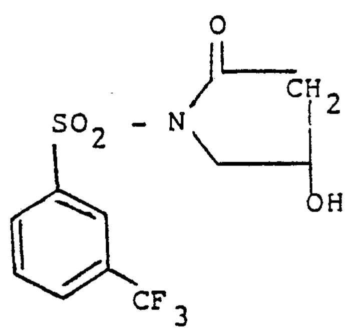

1 419

Synthese des composés entrant dans la composition des médicaments selon l'invention ainsi que des composés nouveaux selon l'invention

Les composés de formule générale (I) peuvent être obtenus par cyclisation du benzènesulfonamidoacide de formule générale (XVII) :

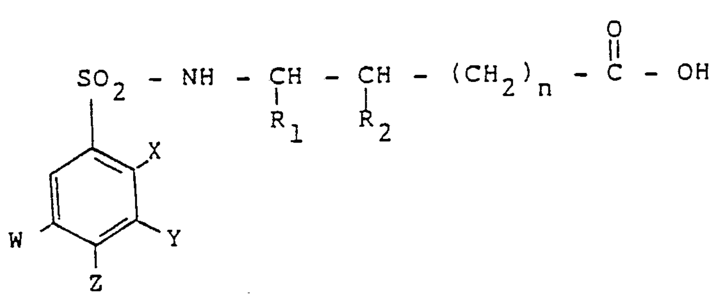

(XVII)

dans laquelle X, Y, Z, W, n, $R_1$ et $R_2$ ont les significations indiquées précédemment.

La cyclisation des benzènesulfonamides peut se faire en utilisant un agent de cyclisation, en milieu acide, et en chauffant. On peut par exemple utiliser comme agent cyclisant $P_2O_5$ et comme acide $H_3PO_4$.

Ce mode d'obtention des composés de formule générale (I) sera désigné par Méthode A.

Les composés selon l'invention susceptibles d'être préparés selon cette méthode A sont ceux dans lesquels n vaut 1, $R_2$ représente un atome d'hydrogène, et X, Y, Z et W sont tous différents d'un groupe alcoxy.

On donne ci-après un exemple général de mise en œuvre de la méthode A pour obtenir les composés de formule générale (I).

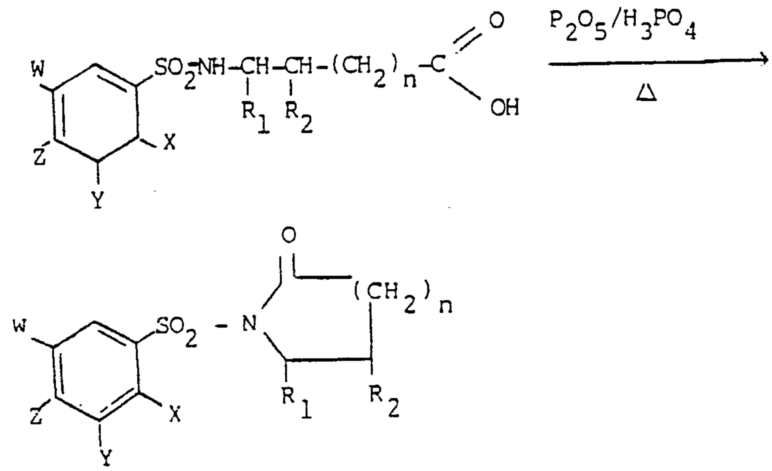


A 10 ml d'acide orthophosphorique à 85 % sont ajoutés, avec précaution, 20 g d'anhydride phosphorique puis 0,01 mole de dérivé benzènesulfonamido-acide. Le mélange réactionnel est porté de 30 minutes à 3 heures à une température comprise entre 40 et 90 °C, selon les produits, puis versé sur de la glace pilée. Les cristaux sont essorés, lavés abondamment à l'eau puis par une solution de bicarbonate de sodium N.

Le produit est alors recristallisé dans les solvants usuels appropriés.

Le rendement est de 60 % à 90 %.

La cyclisation des benzènesulfonamidoacides de formule générale (XVII) peut également se faire avantageusement en ayant recours à de l'anhydride trifluoroacétique comme agent cyclisant, en présence de trifluoroacétate de sodium et en chauffant.

Ce mode d'obtention des composés de formule générale (I) sera désigné par la suite par Méthode B.

Cette méthode B présente l'avantage de pouvoir être utilisée pour la préparation de tous les composés de formule générale (I), et de fournir un bon rendement.

On utilise de préférence la méthode B lorsque $R_2$ représente OH ou $OCOCH_3$.

On donne ci-après un exemple général de mise en œuvre de la méthode B pour obtenir les composés de formule générale (I).

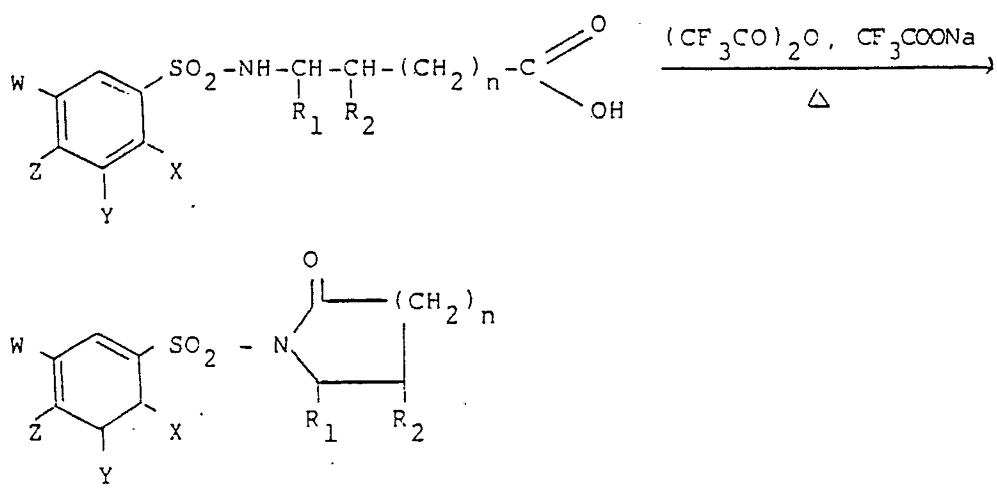


On porte au reflux sous agitation pendant 2 à 24 heures, 0,01 mole de dérivé benzènesulfonamidoacide et 0,5 g de trifluoroacétate de sodium anhydre dans 20 ml d'anhydride trifluoroacétique. Après

évaporation du milieu réactionnel sous vide, le résidu est repris dans 20 ml de méthanol anhydre puis porté 30 minutes au reflux. La solution méthanolique est évaporée à sec, le résidu est repris par 10 ml d'eau.

Les cristaux sont essorés, puis recristallisés dans les solvants organiques appropriés.

Le rendement est de 65 % à 90 %.

Les benzènesulfonamidoacides utilisés sont préparés comme il est indiqué dans les demandes de brevet FR n° 81 11 858 et 81 11 859.

En ce qui concerne les benzènesulfonamidoacides dans lesquels $R_2$ représente OH et n, $R_1$, X, Y, Z, W ont les significations indiquées ci-dessus, ils sont nouveaux.

Ces nouveaux benzènesulfonamidoacides répondent à la formule générale (XVIIa) suivante :

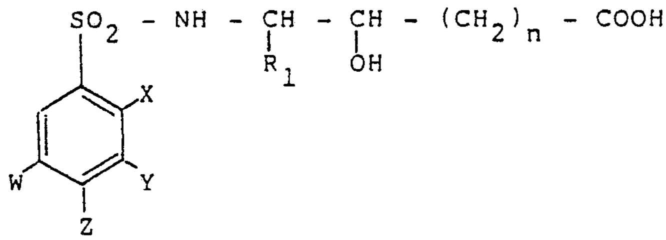

(XVIIa)

Ces composés peuvent être préparés en condensant un sulfohalogénure, notamment un sulfochlorure de formule générale (XVIII) :

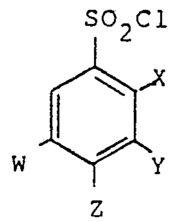

(XVIII)

sur un aminoacide de formule générale (XX) :

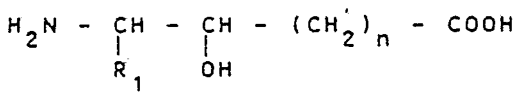

(XX)

selon les procédés tels que ceux décrits dans les demandes de brevet FR n° 81 11 858 et 81 11 859.

On donne ci-après, à titre d'exemple, la préparation du composé de formule :

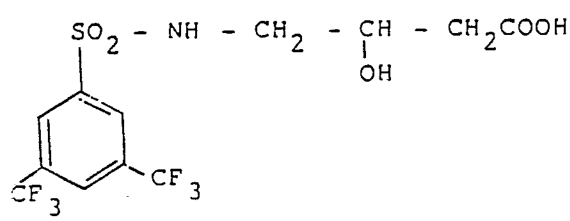

A 3,57 g (0,030 mole) d'acide d, l amino-4-hydroxy-3 butyrique dans 60 ml de soude N, on ajoute goutte à goutte sous agitation vigoureuse une solution de 9,4 g (0,030 mole) de chlorure de ditrifluorométhyle-3,5 benzènesulfonyle dans 60 ml d'éther éthylique.

Après 4 heures d'agitation, la phase organique est éliminée. La phase aqueuse est alors amenée à pH 3 par de l'acide chlorhydrique 2N. Le précipité formé est essoré, lavé à l'eau jusqu'à disparition d'ions chlorure, puis séché.

F = 160 °C Rdt = 58 % p. m. = 395,29

Analyse théorique pour $C_{12}H_{11}F_6NO_5S$

Calculée : C 36,4 H 2,8 N 3,6 % ;

Trouvée : C 36,3 H 3,1 N 3,8 %.

Ont été également préparés par ce procédé les composés de formule :

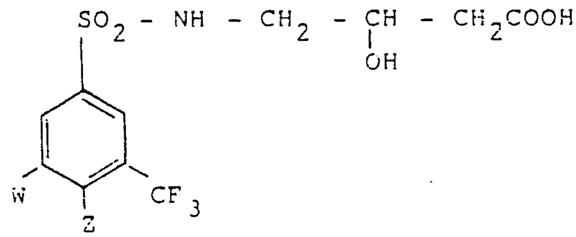

dans laquelle Z représente Cl et W représente H et Z représente H et W représente H.

Les composés de formule générale (I) selon l'invention peuvent être également préparés par condensation des sulfohalogénures, notamment sulfochlorures, de formule général (XVIII) :

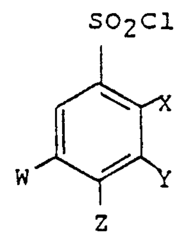

(XVIII)

dans laquelle X, Y, Z et W ont les significations indiquées ci-dessus sur les composés de formule générale (XIX) :

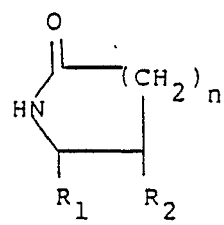

(XIX)

dans laquelle $R_1$, $R_2$ et n ont les significations indiquées ci-dessus.

Cette réaction est de préférence effectuée en présence d'une base forte, pour arracher le proton du composé (XIX), tel que l'hydrure de sodium, dans un solvant tel que le xylène puis sous l'effet du chauffage, la condensation a lieu.

Cette méthode sera désignée ci-après par Méthode C.

Les composés selon l'invention susceptibles d'être préparés par cette méthode sont ceux dans lesquels $R_2$ représente un atome d'hydrogène. On donne ci-après un exemple général de mise en œuvre de la méthode C.

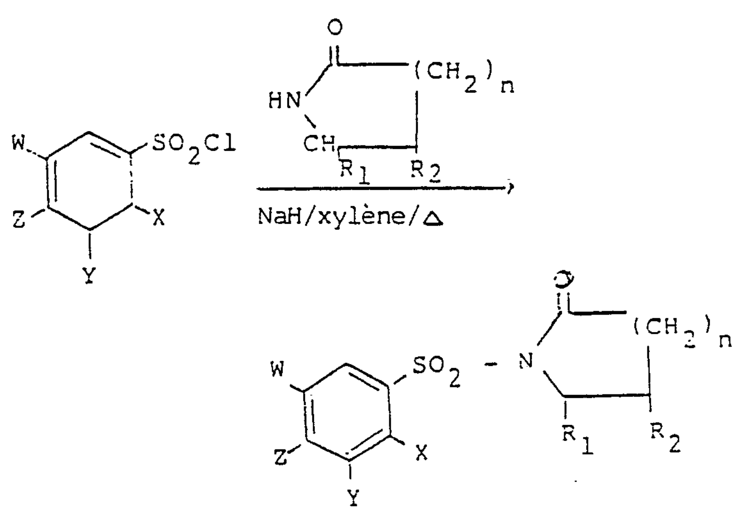


A 0,01 mole d'hydrure de sodium dans 50 ml de xylène anhydre, on ajoute goutte à goutte 0,01 mole de dérivé lactame, sous agitation. On porte le milieu réactionnel pendant 2 heures à 80 °C. Après

refroidissement, on ajoute goutte à goutte 0,01 mole du sulfochlorure approprié et on porte le milieu réactionnel à 110 °C pendant 2 à 8 heures.

Après élimination du solvant par distillation sous vide, le résidu est repris dans l'eau. Le précipité est essoré, lavé à l'eau, puis recristallisé dans les solvants usuels.

Le rendement est de 10 % à 50 %.

Les sulfochlorures utilisés pour la mise en œuvre de la méthode C sont ceux décrits dans la littérature ainsi que dans les brevets et demandes de brevet FR n° 2 313 918, 2 338 929, 2 504 528, 2 504 527.

Les produits qui ont été préparés selon la méthode A sont les suivants : n° 1 043, 1 289, 1 277, 1 276, 1 355, 1 351, 1 416, 1 039.

Les produits qui ont été préparés selon la méthode B sont les suivants : n° 1 043, 1 289, 1 277, 1 276, 1 355, 1 351, 1 416, 1 419, 1 417, 1 418, 1 415, 1 120, 1 287, 1 288, 1 124, 1 285, 1 286, 1 309, 1 332, 1 310, 1 281, 1 282, 1 442, 1 441, 1 039.

Les produits qui ont été préparés selon la méthode C sont les suivants : n° 1 043, 1 289, 1 277, 1 276, 1 355, 1 351, 1 416, 1 120, 1 287, 1 288, 1 124, 1 285, 1 286, 1 309, 1 332, 1 310, 1 281, 1 282, 1 442, 1 441.

Le tableau ci-après rassemble les composés préparés selon les méthodes A, B et C et indique pour chacun d'eux la formule, le point de fusion et le rendement.

L'analyse centésimale des produits obtenus est conforme aux normes habituelles (± 0,3 %).

(Voir Formules Pages 15, 16 et 17)

Formule générale

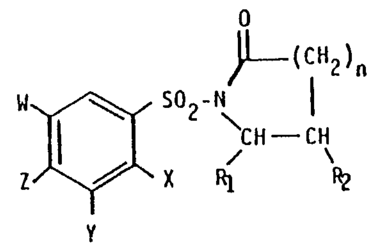

| COMPOSE N° | X | Y | Z | W | n | $R_1$ | $R_2$ | FORMULE BRUTE | P.M. | F°C | Rdt |
|---|---|---|---|---|---|---|---|---|---|---|---|
| 1043 | H | $CF_3$ | H | H | 1 | H | H | $C_{11}H_{10}F_3NO_3S$ | 293,27 | 74 | 85 |
| 1289 | H | $CF_3$ | H | H | 2 | H | H | $C_{12}H_{12}F_3NO_3S$ | 307,30 | 68 | 75 |
| 1277 | H | $CF_3$ | H | H | 3 | H | H | $C_{13}H_{14}F_3NO_3S$ | 321,33 | 78 | 40 |
| 1276 | H | $CF_3$ | H | H | 1 | $CH_3$ | H | $C_{12}H_{12}F_3NO_3S$ | 307,30 | 103 | 35 |
| 1355 | H | $CF_3$ | Cl | H | 1 | H | H | $C_{11}H_9ClF_3NO_3S$ | 327,72 | 118 | 91 |
| 1351 | H | $CF_3$ | H | $CF_3$ | 1 | H | H | $C_{12}H_9F_6NO_3S$ | 361,26 | 121 | 76 |
| 1416 | H | $CF_3$ | H | $CF_3$ | 2 | H | H | $C_{13}H_{11}F_6NO_3S$ | 375,30 | 140 | 86 |
| 1419 | H | $CF_3$ | H | H | 1 | H | OH | $C_{11}H_{10}F_3NO_4S$ | 309,27 | 100 | 64 |

| Composé N° | X | Y | Z | W | n | $R_1$ | $R_2$ | FORMULE BRUTE | P.M. | F°C | Rdt |
|---|---|---|---|---|---|---|---|---|---|---|---|
| 1417 | H | $CF_3$ | Cl | H | 1 | H | OH | $C_{11}H_9ClF_3NO_4S$ | 343,72 | 126 | 70 |
| 1418 | H | $CF_3$ | H | $CF_3$ | 1 | H | OH | $C_{12}H_9F_6NO_4S$ | 377,26 | 120 | 66 |
| 1415 | H | $CF_3$ | H | $CF_3$ | 1 | H | $OCOCH_3$ | $C_{14}H_{11}F_6NO_5S$ | 419,31 | 105 | 75 |
| 1120 | H | H | $OCH_3$ | H | 1 | H | H | $C_{11}H_{13}NO_4S$ | 255,30 | 134 | 75 |
| 1287 | H | H | $OCH_3$ | H | 2 | H | H | $C_{12}H_{15}NO_4S$ | 269,32 | 105 | 60 |
| 1288 | H | H | $OCH_3$ | H | 1 | $CH_3$ | H | $C_{12}H_{15}NO_4S$ | 269,32 | 113 | 45 |
| 1124 | $OCH_3$ | H | H | Cl | 1 | H | H | $C_{11}H_{12}ClNO_4S$ | 289,75 | 148 | 50 |
| 1285 | $OCH_3$ | H | H | Cl | 1 | $CH_3$ | H | $C_{12}H_{14}ClNO_4S$ | 303,77 | 126 | 45 |
| 1286 | $OCH_3$ | H | H | Cl | 3 | H | H | $C_{13}H_{16}ClNO_4S$ | 317,80 | 160 | 30 |
| 1309 | $OCH_3$ | H | $OCH_3$ | $OCH_3$ | 1 | H | H | $C_{13}H_{17}NO_6S$ | 315,35 | 168 | 45 |
| 1332 | $OCH_3$ | H | $OCH_3$ | $OCH_3$ | 1 | $CH_3$ | H | $C_{14}H_{19}NO_6S$ | 329,38 | 147 | 40 |

| Composé n° | X | Y | Z | W | n | $R_1$ | $R_2$ | FORMULE BRUTE | P.M. | F°C | Rdt |
|---|---|---|---|---|---|---|---|---|---|---|---|
| 1310 | $OCH_3$ | H | $OCH_3$ | Cl | 1 | H | H | $C_{12}H_{14}ClNO_5S$ | 319,77 | 173 | 60 |
| 1281 | $OCH_3$ | H | $OCH_3$ | Cl | 1 | $CH_3$ | H | $C_{13}H_{16}ClNO_5S$ | 333,80 | 152 | 55 |
| 1282 | $OCH_3$ | H | $OCH_3$ | Cl | 3 | H | H | $C_{14}H_{18}ClNO_5S$ | 347,83 | 160 | 60 |
| 1 458 | $OCH_3$ | H | $OCH_3$ | H | 1 | $CH_3$ | H | $C_{13}H_{17}NO_5S$ | 299,35 | 151 | 45 |
| 1 459 | $OCH_3$ | H | $OCH_3$ | H | 1 | H | H | $C_{12}H_{15}NO_5S$ | 285,32 | 137 | 40 |
| 1 039 | H | $NO_2$ | H | H | 1 | H | H | $C_{10}H_{10}N_2O_5S$ | 270,27 | 173 | 85 |
| | | | | | | | | | | | |
| | | | | | | | | | | | |
| | | | | | | | | | | | |
| | | | | | | | | | | | |
| | | | | | | | | | | | |

Les composés selon l'invention présentent de remarquables propriétés pharmacologiques.

Les composés selon l'invention présentent des propriétés psychotropes avec modification du comportement.

Ces propriétés pharmacologiques ont été étudiées en prenant comme substance de référence l'aniracetam.

Les composés selon l'invention sont avantageusement introduits comme substance active, notamment dans le traitement des troubles de la mémoire, de l'amnésie, dans le cas d'efforts physiques et intellectuels liés à des périodes de surmenage, dans le traitement des troubles du comportement et de l'adaptation.

Les composés selon l'invention se distinguent par le fait qu'ils agissent sur la mémoire et l'attention, augmentent l'activité mentale et équilibrent le comportement émotionnel.

Les composés selon l'invention sont à cet effet conditionnés avec les excipients et adjuvants traditionnels, notamment ceux utilisés pour la préparation des comprimés, poudres, gélules, ampoules buvables, solutions buvables, solutés injectables.

L'administration des médicaments contenant les composés selon l'invention est effectuée de préférence par voie orale, et les doses de composés administrés sont comprises de 50 à 3 000 mg/jour, de préférence de 200 à 2 000 mg/jour, par exemple de 100 à 1 000 mg/jour, en une ou plusieurs fois.

Un exemple de composition pharmaceutique avantageuse est constitué par une gélule ou un comprimé correspondant à une unité de prise en substance active de 25 à 200 mg, de préférence 100 mg, avec des excipients appropriés.

Un autre exemple de composition pharmaceutique avantageuse est constitué par une solution buvable, compatible avec des unités de prise de 25 à 200 mg de substance active.

I Etude relative à la toxicité-

Les composés selon l'invention sont dépourvus de toxicité.

En effet, la valeur de la $DL_{50}$ obtenue avec les composés n° 1 039, 1 043, 1 276, 1 288, administrés par voie intrapéritonéale chez la souris, est supérieure à 1 g/kg, ce qui montre qu'aux doses d'administration, les composés de l'invention ne sont pas toxiques.

II Etude sur le système nerveux central

On a recours à un dispositif constitué d'une cage bipartite, formée d'un compartiment éclairé et d'un compartiment sombre, séparés par une cloison percée d'un passage. Le plancher du secteur sombre est formé par une grille électrifiable.

Après familiarisation avec le dispositif, des souris albinos subissent une à une la séance d'initiation : chaque souris est déposée dans le compartiment éclairé : on mesure sa latence spontanée à entrer dans le compartiment sombre (préféré spontanément) : dès qu'elle est entrée, elle y reçoit un choc électrique.

Après 10 minutes (protocole A), l'animal est replacé dans le compartiment clair.

L'effet est apprécié par l'augmentation de la latence à entrer dans le compartiment sombre, ou le temps passé dans ce même secteur et l'étude de son comportement dans chaque secteur.

Les produits sont administrés par voie IP à 50 mg/kg dans de la gomme arabique à 2 %, 20 minutes avant (protocole A) la séance d'initiation.

Le placébo est constitué par de la gomme arabique à 2 %.

Les résultats relatifs au protocole A sont donnés dans le tableau 2.

(Voir Tableau 2 Page 19)

Tableau 2 - Protocole A

| | Nombre d'animaux | Latences d'entrée dans le secteur sombre lors de la séance d'initiation | Temps passé dans le secteur sombre pendant les 3 min. de la séance de rétention |
|---|---|---|---|
| PLACEBO | 17 | 29,1 ± 6,1 | 20,6 ± 7,9 |
| n° 1039 | 16 | 40,9 ± 8,6 | 38,7 ± 7,9 |
| n° 1043 | 19 | 54,6 ± 8,4 | 30,3 ± 9,5 |
| n° 1120 | 17 | 34,5 ± 5,9 | 24,5 ± 9,8 |
| n° 1351 | 18 | 59,6 ± 12,2 | 30,1 ± 9,2 |
| n° 1355 | 18 | 45,11 ± 7,8 | 33,9 ± 9,3 |
| ANIRACETAM | 16 | 45,2 ± 9,2 | 22,8 ± 9,4 |

L'étude du comportement des souris montre que leur capacité exploratoire est augmentée dans les deux secteurs, ce qui se traduit par une augmentation de la latence d'entrée dans le secteur sombre et une augmentation du temps passé dans le secteur sombre.

III Etude de l'activité motrice au moyen du test dénommé « test d'open field »

Il s'agit d'un test préconisé par Janssen et col. (« Psychopharmacologia », 1960, 1, p. 389-392) amélioré pour la mesure de l'activité motrice par un appareil automatisé par Delbene et col. (« Psychologia », 1970, 18, p. 227-230).

Ce test a été adapté par G. Narcisse et col. (travaux non publiés).

Les doses sont de 50 et 100 mg/kg par voie intrapéritonéale.

Ce test effectué avec le composé n° 1 039 montre que ce composé présente des propriétés psychotropes. Ce test, réalisé dans les mêmes conditions avec les composés de l'invention n° 1 043, 1 276, 1 277, 1 281, 1 288, 1 309, 1 417, 1 418 et 1 419, montre que ces composés présentent également une activité psychotrope.

**Revendications**

1. Composés répondant à la formule générale (I) suivante :

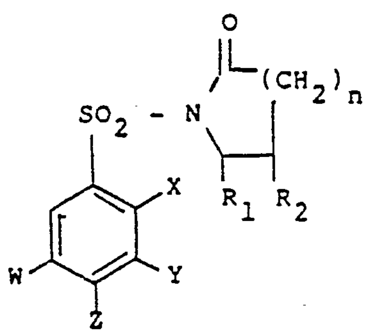

(I)

dans laquelle

— X représente un atome d'hydrogène, un groupe alcoxy de 1 à 4 atomes de carbone ;

— Y représente un atome d'hydrogène, un groupe $CF_3$, $NO_2$ ;

— Z représente un atome d'hydrogène, un atome d'halogène, un groupe alcoxy de 1 à 4 atomes de carbone, un groupe $NO_2$ ;

— W représente un atome d'hydrogène, un atome d'halogène, un groupe $CF_3$, $NO_2$, un groupe alcoxy de 1 à 4 atomes de carbone ;

— n vaut 1, 2 ou 3 ;

— $R_1$ représente un atome d'hydrogène, un groupe alcoyle de 1 à 6 atomes de carbone ;

— $R_2$ représente un atome d'hydrogène, le groupe OH, un groupe OR dans lequel R représente un groupe alcoyle de 1 à 4 atomes de carbone ou un groupe acyle de 1 à 6 atomes de carbone ;

sous réserve que :

— lorsque n vaut 1,

Y ou W représente le groupe $CF_3$

— et lorsque n vaut 2 ou 3,

X ou Z représente un groupe alcoxy de 1 à 4 atomes de carbone ou bien Y ou W représente le groupe $CF_3$ ou $NO_2$.

2. Composés selon la revendication 1, caractérisés en ce qu'ils répondent à la formule générale (I) dans laquelle $R_1$ représente H ou $CH_3$.

3. Composés selon la revendication 1 ou 2, caractérisés en ce qu'ils répondent à la formule générale (I) dans laquelle $R_2$ représente H, OH ou $OCOCH_3$.

4. Composés selon l'une quelconque des revendications 1 à 3, caractérisés en ce qu'ils répondent à la formule suivante :

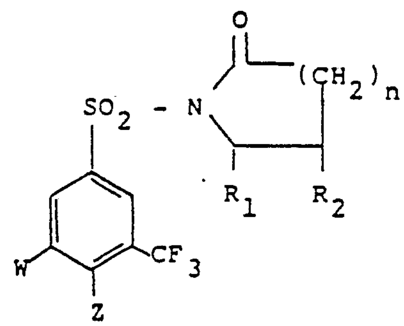

dans laquelle n, $R_1$, $R_2$ ont les significations indiquées à la revendication 1, Z représente H ou Cl et W représente H ou $CF_3$.

5. Composés selon l'une quelconque des revendications 1 à 3, caractérisés en ce qu'ils répondent à la formule suivante :

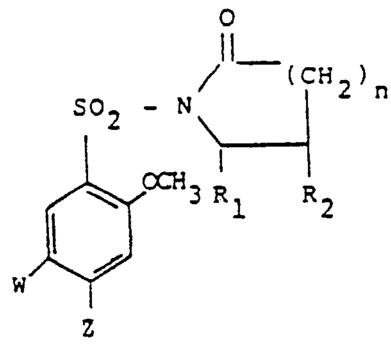

dans laquelle n vaut 2 ou 3, $R_1$ et $R_2$ ont les significations indiquées à la revendication 1 et Z représente H ou $OCH_3$ et W représente H, Cl ou $OCH_3$.

6. Composés selon l'une quelconque des revendications 1 à 3, caractérisés en ce qu'ils répondent à la formule suivante :

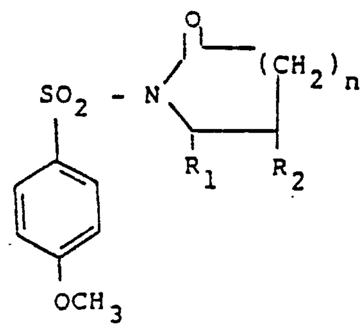

dans laquelle n vaut 2 ou 3, $R_1$ et $R_2$ ont les significations indiquées dans les revendications 1 à 3.

7. Composés selon l'une quelconque des revendications 1 à 3, caractérisés en ce qu'ils répondent à la formule suivante :

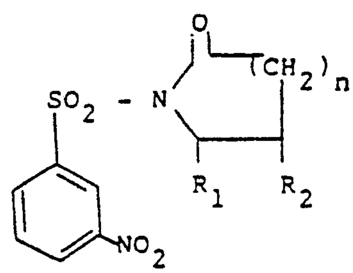

dans laquelle n vaut 2 ou 3, $R_1$ et $R_2$ ont les significations indiquées dans les revendications 1 à 3.

8. Composés selon la revendication 1, caractérisés en ce qu'ils répondent à l'une des formules suivantes :

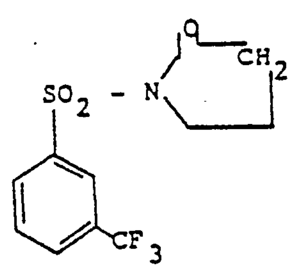

(Suite)

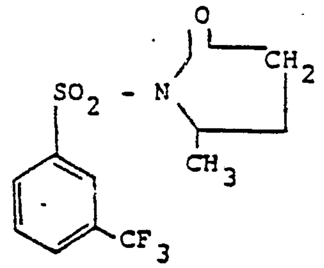

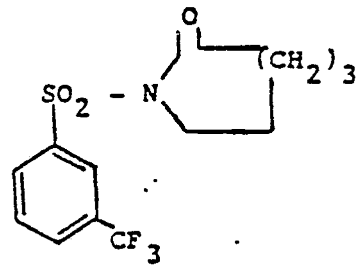

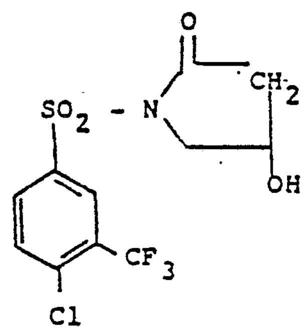

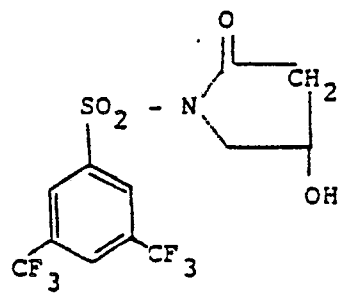

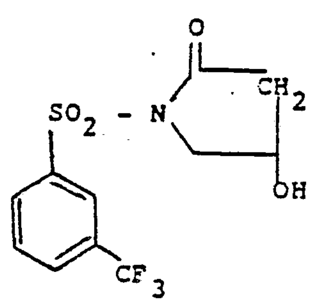

9. Procédé de préparation des composés selon l'une quelconque des revendications 1 à 8, dans lesquels n vaut 1, $R_2$ représente un atome d'hydrogène, X, Y, Z et W ont les significations indiquées aux revendications 1 à 8, mais sont tous différents d'un radical alcoxy, $R_1$ a la signification indiquée aux revendications 1 à 7, caractérisés en ce qu'ils sont obtenus par cyclisation de :

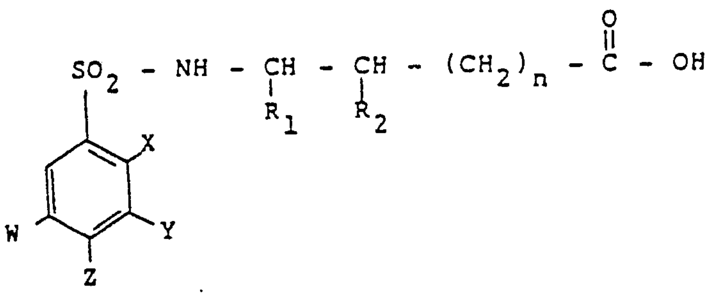

à l'aide d'un agent cyclisant tel que $P_2O_5$, en milieu acide, tel qu'en milieu d'$H_3PO_4$ et en chauffant.

10. Procédé de préparation des composés selon l'une quelconque des revendications 1 à 8, caractérisés en ce qu'ils sont obtenus par cyclisation de :

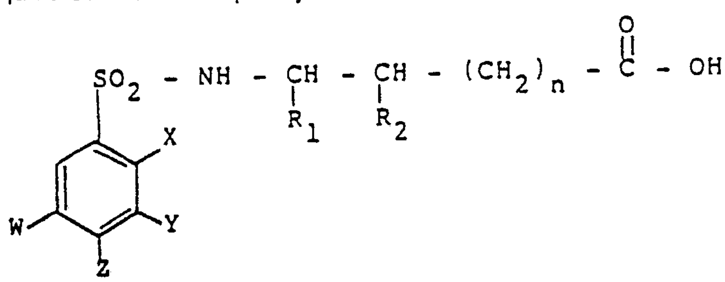

dans laquelle n, $R_1$, $R_2$, X, Y, Z et W ont les significations indiquées aux revendications 1 à 8, à l'aide d'un agent cyclisant tel que l'anhydride trifluoroacétique, en présence de trifluoroacétate de sodium et en chauffant.

11. Procédé de préparation des composés selon l'une quelconque des revendications 1 à 8, dans lesquels $R_2$ représente un atome d'hydrogène, $R_1$, X, Y, Z et W ont les significations indiquées aux revendications 1 à 8, caractérisé en ce que l'on condense le composé de formule :

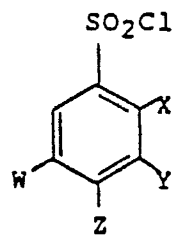

sur le composé de formule :

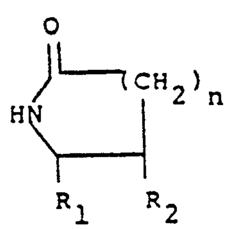

en présence d'une base forte, telle que l'hydrure de sodium, dans un solvant, tel que le xylène et que l'on chauffe.

12. Composition pharmaceutique, caractérisée en ce qu'elle contient, en association avec un véhicule pharmaceutiquement acceptable, l'un au moins des composés de formule générale (I) suivante en tant que substance active :

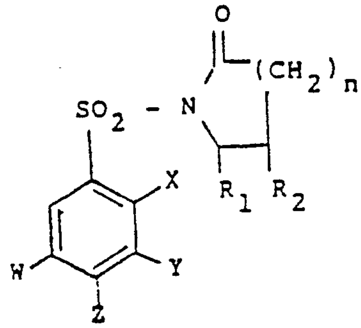

(I)

formule dans laquelle :

— X représente un atome d'hydrogène, un groupe alcoxy de 1 à 4 atomes de carbone ;
— Y représente un atome d'hydrogène, un groupe $CF_3$, $NO_2$ ;
— Z représente un atome d'hydrogène, un atome d'halogène, un groupe alcoxy de 1 à 4 atomes de carbone, un groupe $NO_2$ ;
— W représente un atome d'hydrogène, un atome d'halogène, un groupe $CF_3$, $NO_2$, un groupe alcoxy de 1 à 4 atomes de carbone ;
— n vaut 1, 2 ou 3 ;
— $R_1$ représente un atome d'hydrogène, un groupe alcoyle de 1 à 6 atomes de carbone ;
— $R_2$ représente un atome d'hydrogène, le groupe OH, un groupe OR dans lequel R représente un groupe alcoyle de 1 à 4 atomes de carbone ou un groupe acyle de 1 à 6 atomes de carbone ;

sous réserve que X ou Z représente un groupe alcoxy de 1 à 4 atomes de carbone ou que Y ou W représente le groupe $CF_3$ ou $NO_2$.

13. Composition pharmaceutique selon la revendication 12, caractérisée en ce qu'elle contient un composé de formule générale (I) :

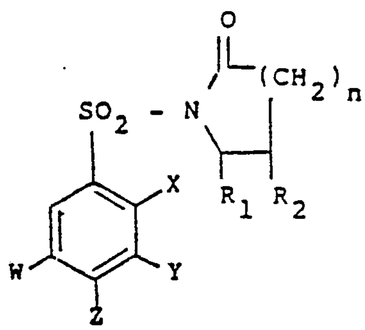

(I)

dans laquelle :

— X représente un atome d'hydrogène, un groupe alcoxy de 1 à 4 atomes de carbone ;
— Y représente un atome d'hydrogène, un groupe $CF_3$, $NO_2$ ;
— Z représente un atome d'hydrogène, un atome d'halogène, un groupe alcoxy de 1 à 4 atomes de carbone, un groupe $NO_2$ ;
— W représente un atome d'hydrogène, un atome d'halogène, un groupe $CF_3$, $NO_2$, un groupe alcoxy de 1 à 4 atomes de carbone ;
— n vaut 1, 2 ou 3 ;
— $R_1$ représente un atome d'hydrogène, un groupe alcoyle de 1 à 6 atomes de carbone ;
— $R_2$ représente un atome d'hydrogène, le groupe OH, un groupe OR dans lequel R représente un groupe alcoyle de 1 à 4 atomes de carbone ou un groupe acyle de 1 à 6 atomes de carbone ;

sous réserve que :

— lorsque n vaut 1,
Y ou W représente le groupe $CF_3$
— et lorsque n vaut 2 ou 3,
X ou Z représente un groupe alcoxy de 1 à 4 atomes de carbone ou bien Y ou W représente le groupe $CF_3$ ou $NO_2$.

14. Composition pharmaceutique selon les revendications 12 ou 13, caractérisée en ce qu'elle contient un composé selon l'une quelconque des revendications 2 à 4.

15. Composition selon l'une quelconque des revendications 12 ou 13, caractérisée en ce qu'elle contient un composé répondant à la formule générale suivante :

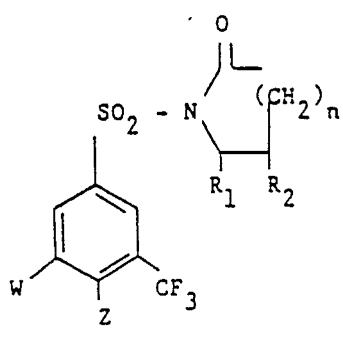

dans laquelle n, $R_1$ et $R_2$ ont les significations indiquées dans la revendication 1, Z représente H ou Cl et W représente H ou $CF_3$.

16. Composition selon l'une quelconque des revendications 12 ou 13, caractérisée en ce qu'elle contient un composé répondant à la formule générale suivante :

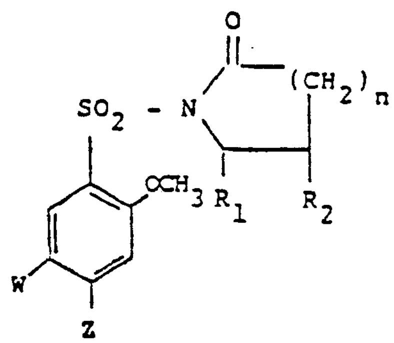

dans laquelle n, $R_1$ et $R_2$ ont les significations indiquées à la revendication 1 et Z représente H ou $OCH_3$ et W représente H, Cl ou $OCH_3$.

17. Composition selon l'une quelconque des revendications 12 ou 13, caractérisée en ce qu'elle contient un composé répondant à la formule générale suivante :

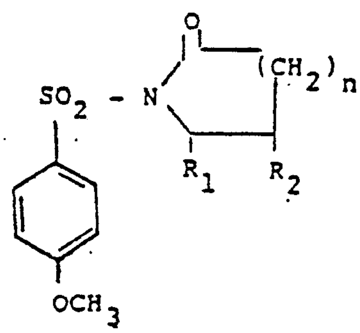

dans laquelle n, $R_1$ et $R_2$ ont les significations indiquées dans les revendications 1 à 3.

18. Composition selon l'une quelconque des revendications 12 ou 13, caractérisée en ce qu'elle contient un composé répondant à la formule générale suivante :

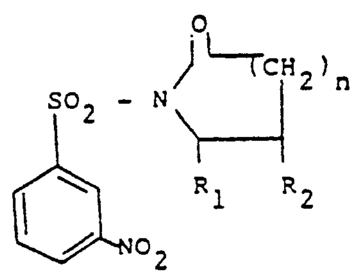

dans laquelle n, $R_1$ et $R_2$ ont les significations indiquées dans les revendications 1 à 3.

19. Composition pharmaceutique selon l'une quelconque des revendications 12 à 18 présentant une action psychotrope, caractérisée en ce qu'elle est conditionnée pour être administrée à des doses de 100 à 1 000 mg de substance active par jour.

20. Composition pharmaceutique selon l'une quelconque des revendications 12 à 18, caractérisée en ce qu'elle se présente sous forme d'un conditionnement unitaire constitué par une gélule ou un comprimé dosé à raison de 25 à 200 mg de substance active.

21. Composition pharmaceutique selon l'une quelconque des revendications 12 à 18, caractérisée en ce qu'elle se présente sous forme d'un conditionnement unitaire constitué par une solution buvable compatible avec des unités de prise de 25 à 200 mg de substance active.

**Claims**

1. Compounds corresponding to the following general formula (I) :

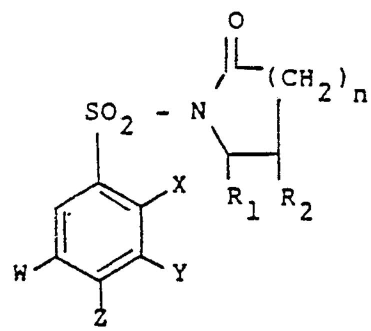

(I)

in which :

— X represents a hydrogen atom, an alkoxy group from 1 to 4 carbon atoms ;

— Y represents a hydrogen atom, a $CF_3$ group, a $NO_2$ group ;

— Z represents a hydrogen atom, a halogen atom, an alkoxy group from 1 to 4 carbon atoms, a $NO_2$ group ;

— W represents a hydrogen atom, a halogen atom, a $CF_3$ group, a $NO_2$ group, an alkoxy group from 1 to 4 carbon atoms ;

— n is 1, 2 or 3 ;

— $R_1$ represents a hydrogen atom, an alkyl group from 1 to 6 carbon atoms ;

— $R_2$ represents a hydrogen atom, an OH group, an OR group in which R represents an alkyl group from 1 to 4 carbon atoms or an acyl group from 1 to 6 carbon atoms ;

provided that when n is 1 :

— Y or W represents a $CF_3$ group ; and

when n is 2 or 3 :

— X or Z represents an alkoxy group from 1 to 4 carbon atoms or Y or W represents the $CF_3$ or $NO_2$ group.

2. Compounds according to claim 1, characterized by the fact that they correspond to general formula (I) in which $R_1$ represents H or $CH_3$.

3. Compounds according to claim 1 or 2, characterized by the fact that they correspond to the general formula (I) in which $R_2$ represents H, OH or $OCOCH_3$.

4. Compounds according to anyone of claims 1 to 3, characterized by the fact that they correspond to the following formula :

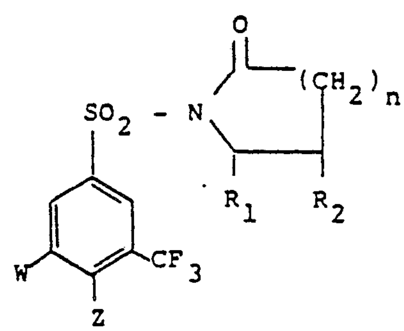

in which n, $R_1$, $R_2$ have the meanings indicated in claim 1, Z represents H or Cl and W represents H or $CF_3$.

5. Compounds according to anyone of claims 1 to 3, characterized by the fact that they correspond to the following formula :

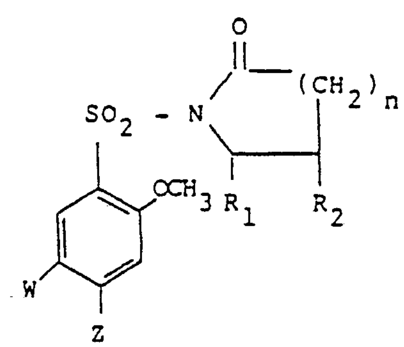

wherein n is 2 or 3, $R_1$ and $R_2$ have the meanings indicated in claim 1 and Z represents H or $OCH_3$ and W represents H, Cl or $OCH_3$.

6. Compounds according to anyone of claims 1 to 3, characterized by the fact that they correspond to the following formula :

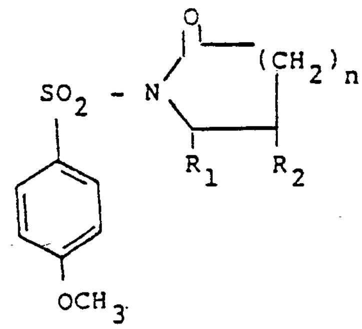

wherein n is 2 or 3, $R_1$ and $R_2$ have the meanings indicated in claims 1 to 3.

7. Compounds according to anyone of claims 1 to 3, characterized by the fact that they correspond to the following formula :

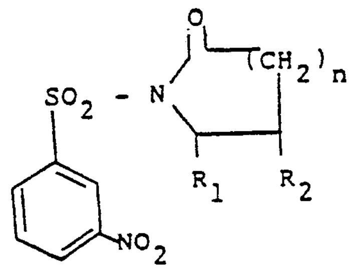

wherein n is 2 or 3, $R_1$ and $R_2$ have the meanings indicated in claims 1 to 3.

8. Compounds according to claim 1, characterized by the fact that they correspond to one of the following formulas :

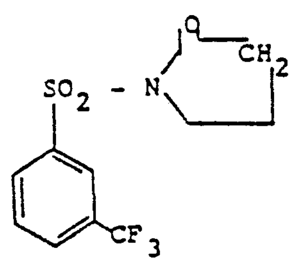

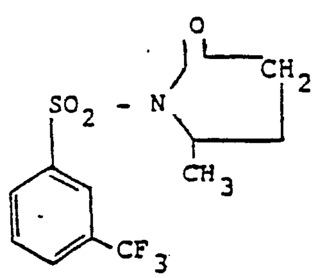

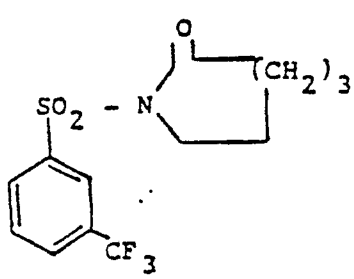

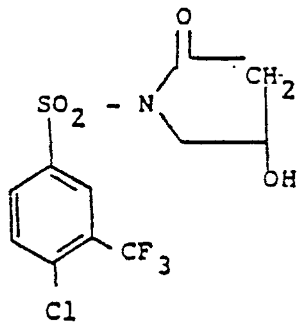

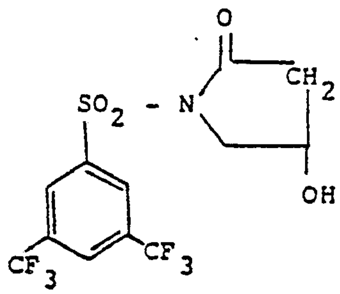

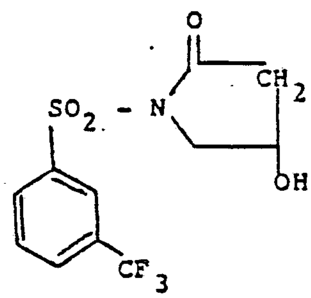

9. Process for preparing the compounds according to anyone of claims 1 to 8, wherein n is 1, $R_2$ represents a hydrogen atom, X, Y, Z and W have the meanings indicated in claims 1 to 8, but are all different from an alkoxy radical, $R_1$ has the meaning indicated in claims 1 to 7, said process comprising the cyclization of :

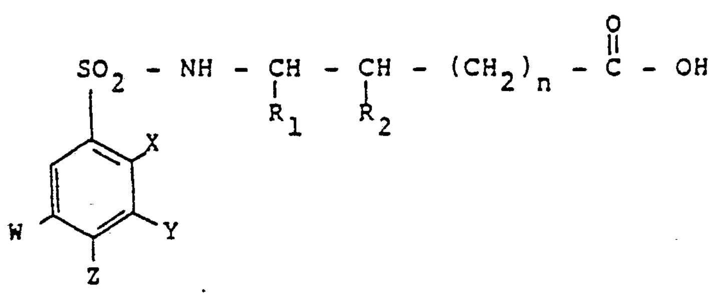

by means of a cyclizing agent such as $P_2O_5$, in an acid medium, such as in a $H_3PO_4$ medium and by heating.

10. Process for preparing compounds according to anyone of claims 1 to 8, characterized in that they are obtained by cyclization of :

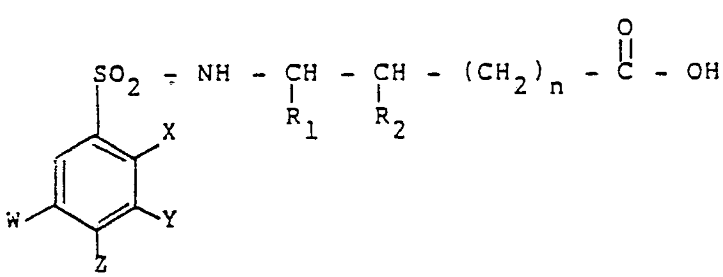

in which n, $R_1$, $R_2$, X, Y, Z and W have the meanings indicated in claims 1 to 8, by means of a cyclizing agent such as trifluoroacetic anhydride, in the presence of sodium trifluoroacetate and by heating.

11. Process for preparing compounds according to anyone of claims 1 to 8, wherein $R_2$ represents a hydrogen atom, $R_1$, X, Y, Z and W have the meanings indicated in claims 1 to 8, characterized in that the compound of formula :

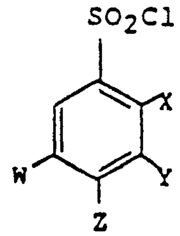

is condensed with the compound of the formula :

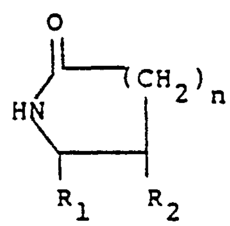

in the presence of a strong base, such as sodium hydrid, in a solvent such as xylene and by heating.

12. Pharmaceutical composition, characterized by the fact that it contains, in association with a pharmaceutically acceptable vehicle, one at least of the compounds of the following general formula (I) as active substance :

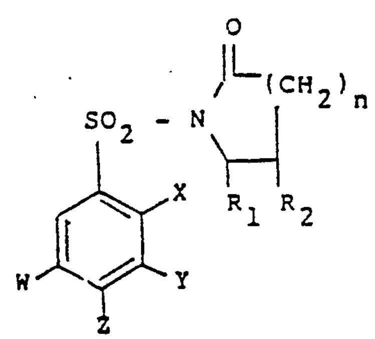

(I)

in which :

— X represents a hydrogen atom, an alkoxy group from 1 to 4 carbon atoms ;

— Y represents a hydrogen atom, a $CF_3$ group, a $NO_2$ group ;

— Z represents a hydrogen atom, a halogen atom, an alkoxy group from 1 to 4 carbon atoms, a $NO_2$ group ;

— W represents a hydrogen atom, a halogen atom, a $CF_3$ group, a $NO_2$ group, an alkoxy group from 1 to 4 carbon atoms ;

— n is 1, 2 or 3 ;

— $R_1$ represents a hydrogen atom, an alkyl group from 1 to 6 carbon atoms ;

— $R_2$ represents a hydrogen atom, an OH group, an OR group in which R represents an alkyl group from 1 to 4 carbon atoms or an acyl group from 1 to 6 carbon atoms ;

provided that X or Z represents an alkoxy group from 1 to 4 carbon atoms or Y or W represents the $CF_3$ or $NO_2$ group.

13. Pharmaceutical composition according to claim 12, characterized by the fact that it contains a compound of general formula (I) :

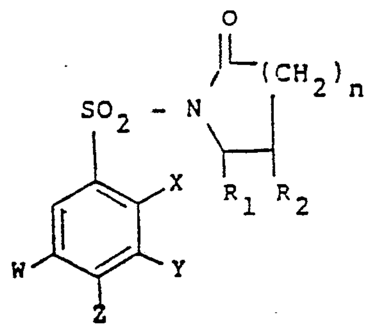

(I)

in which :

— X represents a hydrogen atom, an alkoxy group from 1 to 4 carbon atoms ;

— Y represents a hydrogen atom, a $CF_3$ group, a $NO_2$ group ;

— Z represents a hydrogen atom, a halogen atom, an alkoxy group from 1 to 4 carbon atoms, a $NO_2$ group ;

— W represents a hydrogen atom, a halogen atom, a $CF_3$ group, a $NO_2$ group, an alkoxy group from 1 to 4 carbon atoms ;

— n is 1, 2 or 3 ;

— $R_1$ represents a hydrogen atom, an alkyl group from 1 to 6 carbon atoms ;

— $R_2$ represents a hydrogen atom, an OH group, an OR group in which R represents an alkyl group from 1 to 4 carbon atoms or an acyl group from 1 to 6 carbon atoms ;

provided that when n is 1 :

— Y or W represents a $CF_3$ group ; and

when n is 2 or 3 :

— X or Z represents an alkoxy group from 1 to 4 carbon atoms or Y or W represents the $CF_3$ or $NO_2$ group.

14. Pharmaceutical composition according to claims 12 or 13, characterized by the fact that it contains a compound according to anyone of claims 2 to 4.

15. Pharmaceutical composition according to claims 12 or 13, characterized by the fact that it contains a compound of the following general formula :

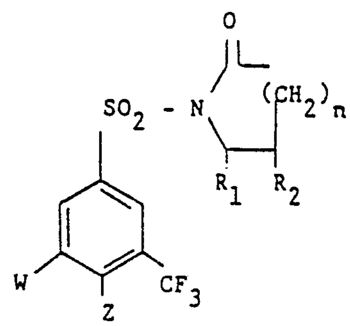

in which n, $R_1$, $R_2$ have the meaning indicated in claim 1, Z represents H or Cl and W represents H or $CF_3$.

16. Composition according to anyone of claims 12 or 13, characterized by the fact that it contains a compound of the following general formula :

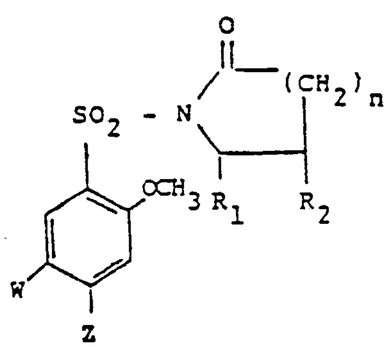

wherein n, $R_1$ and $R_2$ have the meanings indicated in claim 1 and Z represents H or $OCH_3$ and W represents H, Cl or $OCH_3$.

17. Composition according to anyone of claims 12 or 13, characterized by the fact that it contains a compound of the following general formula :

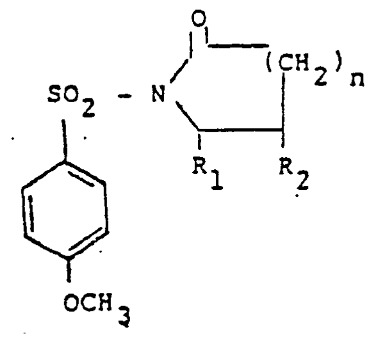

wherein n, $R_1$ and $R_2$ have the meanings mentioned in claims 1 to 3.

18. Process according to anyone of claims 12 or 13, characterized by the fact that it contains a compound of the following general formula :

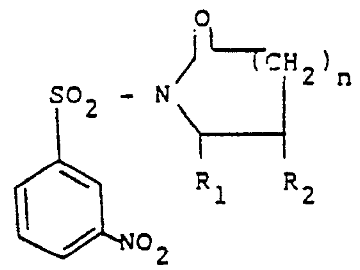

wherein n, $R_1$ and $R_2$ have the meanings indicated in claims 1 to 3.

19. Pharmaceutical composition according to anyone of claims 12 to 18, presenting a psychotrope activity, characterized by the fact that it is packaged to be administered at doses of 100 to 1 000 mg of active substance a day.

20. Pharmaceutical composition according to anyone of claims 12 to 18, characterized by the fact that it is under the unit form packaging constituted by a capsule or a tablet of 25 to 200 mg of active substance.

21. Pharmaceutical composition according to anyone of claims 12 to 18, characterized by the fact that it is under the unit form packaging constituted by a drinkable solution suitable for unit dosage of 25 to 200 mg of active substance.

**Patentansprüche**

1. Verbindungen der allgemeinen Formel (I)

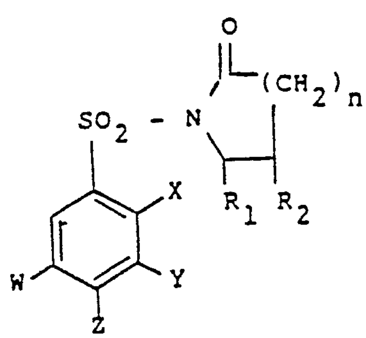

(I)

worin

X ein Wasserstoffatom oder eine Alkoxygruppe mit 1 bis 4 Kohlenstoffatomen darstellt ;

Y ein Wasserstoffatom, eine $CF_3$ Gruppe oder eine $NO_2$ Gruppe darstellt ;

Z ein Wasserstoffatom, ein Halogenatom, eine Alkoxygruppe mit 1 bis 4 Kohlenstoffatomen oder eine $NO_2$ Gruppe darstellt ;

W ein Wasserstoffatom, ein Halogenatom, eine $CF_3$ Gruppe, eine $NO_2$ Gruppe, eine Alkoxygruppe mit 1 bis 4 Kohlenstoffatomen darstellt ;

n 1, 2 oder 3 ist ;

$R_1$ ein Wasserstoffatom oder eine Alkylgruppe mit 1 bis 6 Kohlenstoffatomen darstellt ;

$R_2$ ein Wasserstoffatom, eine OH-Gruppe oder eine OR-Gruppe darstellt, worin R eine Alkylgruppe mit 1 bis 4 Kohlenstoffatomen oder eine Acylgruppe mit 1 bis 6 Kohlenstoffatomen darstellt ; unter der Voraussetzung, daß,

wenn n 1 ist,

Y oder W die $CF_3$ Gruppe darstellen ; und

wenn n 2 oder 3 ist,

X oder Z eine Alkoxygruppe mit 1 bis 4 Kohlenstoffatomen oder

Y oder W die $CF_3$ oder $NO_2$ Gruppe darstellen.

2. Verbindungen nach Anspruch 1, dadurch gekennzeichnet, daß sie der allgemeinen Formel (I) entsprechen, worin $R_1$ H oder $CH_3$ darstellt.

3. Verbindungen nach Anspruch 1 oder 2, dadurch gekennzeichnet, daß sie der allgemeinen Formel (I) entsprechen, worin $R_2$ H, OH oder $OCOCH_3$ darstellt.

4. Verbindungen nach einem der Ansprüche 1 bis 3, dadurch gekennzeichnet, daß sie der Formel

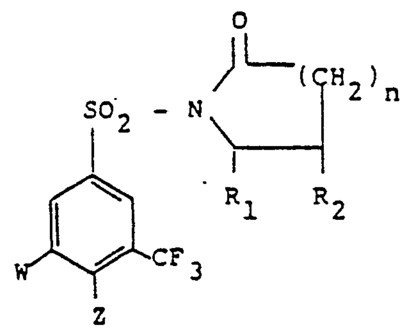

entsprechen, worin n, $R_1$ und $R_2$ die in Anspruch 1 angegebenen Bedeutungen haben, Z H oder Cl darstellt und W H oder $CF_3$ darstellt.

5. Verbindungen nach einem der Ansprüche 1 bis 3, dadurch gekennzeichnet, daß sie der Formel

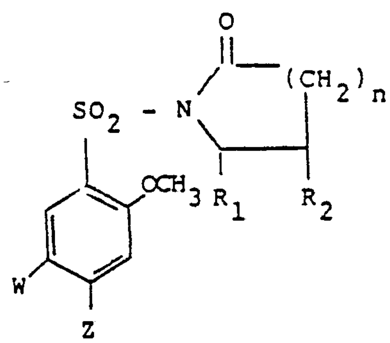

entsprechen, worin n 2 oder 3 ist, $R_1$ und $R_2$ die in Anspruch 1 angegebenen Bedeutungen haben und Z H oder $OCH_3$ darstellt, und W H, Cl oder $OCH_3$ darstellt.

6. Verbindungen nach einem der Ansprüche 1 bis 3, dadurch gekennzeichnet, daß sie der Formel

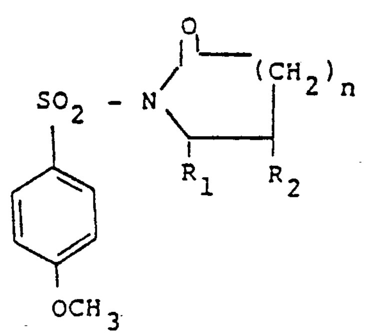

entsprechen, worin n 2 oder 3 ist, und $R_1$ und $R_2$ die in den Ansprüchen 1 bis 3 angegebenen Bedeutungen haben.

7. Verbindungen nach einem der Ansprüche 1 bis 3, dadurch gekennzeichnet, daß sie der Formel

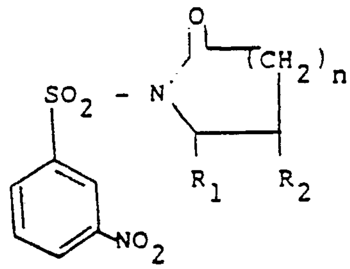

entsprechen, worin n 2 oder 3 ist, und $R_1$ und $R_2$ die in den Ansprüchen 1 bis 3 angegebenen Bedeutungen haben.

8. Verbindungen nach Anspruch 1, dadurch gekennzeichnet, daß sie einer der folgenden Formeln entsprechen :

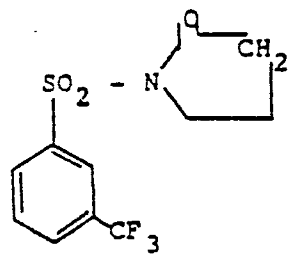

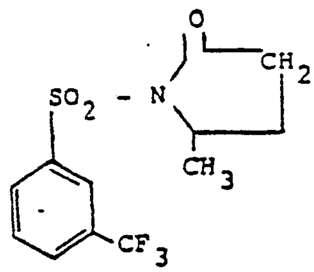

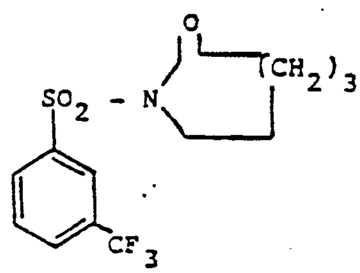

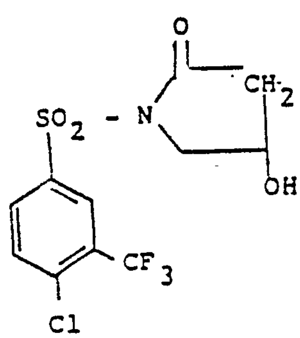

(Fortsetzung)

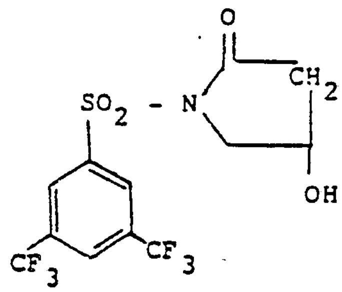

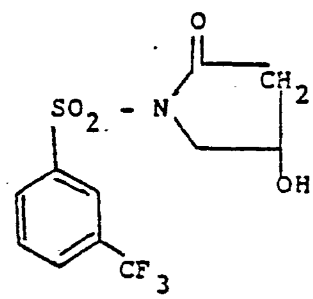

9. Verfahren zur Herstellung der Verbindungen nach einem der Ansprüche 1 bis 8, worin n 1 ist, $R_2$ ein Wasserstoffatom darstellt und X, Y, Z und W die in den Ansprüchen 1 bis 8 angegebenen Bedeutungen haben, sich jedoch von einer Alkoxygruppe unterscheiden, $R_1$ die in den Ansprüchen 1 bis 7 angegebene Bedeutung hat, dadurch gekennzeichnet, daß sie durch die Zyklisierung von

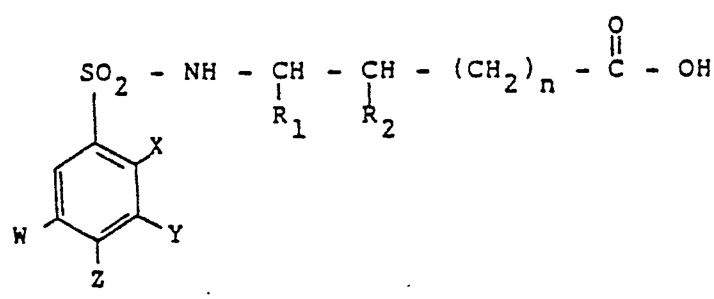

mit Hilfe eines Zyklisierungsmittels, wie $P_2O_5$, in einem sauren Medium, wie in einem $H_3PO_4$ Medium, und durch Erwärmen erhalten werden.

10. Verfahren zur Herstellung der Verbindungen nach einem der Ansprüche 1 bis 8, dadurch gekennzeichnet, daß sie durch die Zyklisierung von

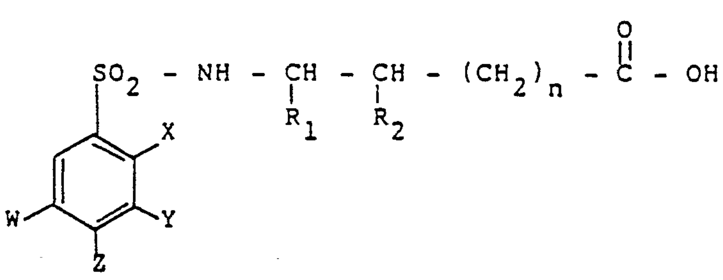

worin n, $R_1$, $R_2$, X, Y, Z und W die in den Ansprüchen 1 bis 8 angegebenen Bedeutungen haben, mit Hilfe eines Zyklisierungsmittels, wie Trifluoracetanhydrid, in Gegenwart von Natriumtrifluoracetat und durch Erwärmen erhalten werden.

11. Verfahren zur Herstellung der Verbindungen nach einem der Ansprüche 1 bis 8, worin $R_2$ ein Wasserstoffatom darstellt, $R_1$, X, Y, Z und W die in den Ansprüchen 1 bis 8 angegebenen Bedeutungen haben, dadurch gekennzeichnet, daß die Verbindung der Formel

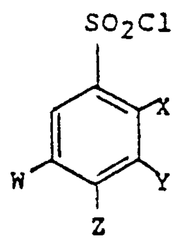

mit der Verbindung der Formel

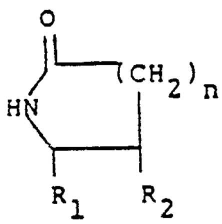

in Gegenwart einer starken Base, wie Natriumhydrid, in einem Lösungsmittel, wie Xylol, und durch Erwärmen kondensiert wird.

12. Pharmazeutische Zusammensetzung, dadurch gekennzeichnet, daß sie, in Verbindung mit einem pharmazeutisch verträglichen Träger, wenigstens eine der Verbindungen der allgemeinen Formel (I) als Wirkstoff enthält

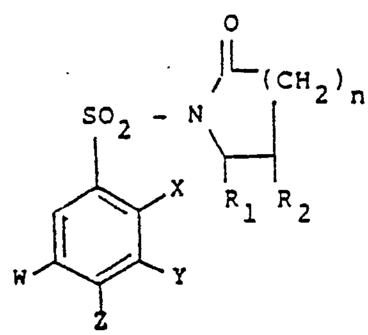

(I)

worin

X ein Wasserstoffatom oder eine Alkoxygruppe mit 1 bis 4 Kohlenstoffatomen darstellt ;

Y ein Wasserstoffatom, eine $CF_3$ Gruppe oder eine $NO_2$ Gruppe darstellt ;

Z ein Wasserstoffatom, ein Halogenatom, eine Alkoxygruppe mit 1 bis 4 Kohlenstoffatomen oder eine $NO_2$ Gruppe darstellt ;

W ein Wasserstoffatom, ein Halogenatom, eine $CF_3$ Gruppe, eine $NO_2$ Gruppe oder eine Alkoxygruppe mit 1 bis 4 Kohlenstoffatomen darstellt ;

n 1, 2 oder 3 ist ;

$R_1$ ein Wasserstoffatom oder eine Alkylgruppe mit 1 bis 6 Kohlenstoffatomen darstellt ;

$R_2$ ein Wasserstoffatom, die OH-Gruppe oder eine OR-Gruppe darstellt, worin R eine Alkylgruppe mit 1 bis 4 Kohlenstoffatomen oder eine Acylgruppe mit 1 bis 6 Kohlenstoffatomen darstellt ; unter der Voraussetzung,

daß X oder Z eine Alkoxygruppe mit 1 bis 4 Kohlenstoffatomen, oder Y oder W die $CF_3$ oder $NO_2$ Gruppe darstellen.

13. Pharmazeutische Zusammensetzung nach Anspruch 12, dadurch gekennzeichnet, daß sie eine Verbindung der allgemeinen Formel (I) enthält,

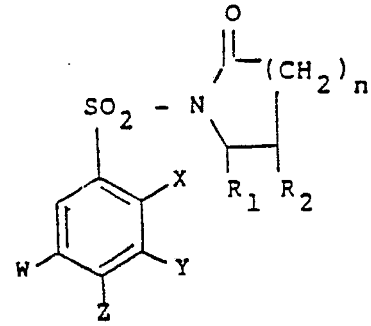

(I)

worin

X ein Wasserstoffatom oder eine Alkoxygruppe mit 1 bis 4 Kohlenstoffatomen darstellt ;

Y ein Wasserstoffatom, eine $CF_3$ Gruppe oder eine $NO_2$ Gruppe darstellt ;

Z ein Wasserstoffatom, ein Halogenatom, eine Alkoxygruppe mit 1 bis 4 Kohlenstoffatomen oder eine $NO_2$ Gruppe darstellt ;

W ein Wasserstoffatom, ein Halogenatom, eine $CF_3$ Gruppe, eine $NO_2$ Gruppe oder eine Alkoxygruppe mit 1 bis 4 Kohlenstoffatomen darstellt ;

n 1, 2 oder 3 ist ;

$R_1$ ein Wasserstoffatom oder eine Alkylgruppe mit 1 bis 6 Kohlenstoffatomen darstellt ;

$R_2$ ein Wasserstoffatom, die OH-Gruppe oder eine OR-Gruppe darstellt, worin R eine Alkylgruppe mit 1 bis 4 Kohlenstoffatomen oder eine Acylgruppe mit 1 bis 6 Kohlenstoffatomen darstellt ; unter der Voraussetzung, daß, wenn n 1 ist,

Y oder W die $CF_3$ Gruppe darstellen ;

und, wenn n 2 oder 3 ist,

X oder Z eine Alkoxygruppe mit 1 bis 4 Kohlenstoffatomen oder Y oder W die $CF_3$ Gruppe oder $NO_2$ Gruppe darstellen.

14. Pharmazeutische Zusammensetzung nach Anspruch 12 oder 13, dadurch gekennzeichnet, daß sie eine Verbindung nach einem der Ansprüche 2 bis 4 enthält.

15. Pharmazeutische Zusammensetzung nach einem der Ansprüche 12 oder 13, dadurch gekennzeichnet, daß sie eine Verbindung der allgemeinen Formel

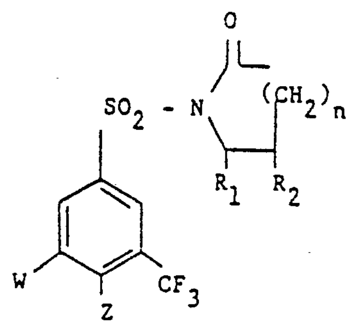

enthält, worin n, $R_1$ und $R_2$ die in Anspruch 1 angegebenen Bedeutungen hat, Z H oder Cl darstellt, und W H oder $CF_3$ darstellt.

16. Zusammensetzung nach einem der Ansprüche 12 oder 13, dadurch gekennzeichnet, daß sie eine Verbindung der allgemeinen Formel

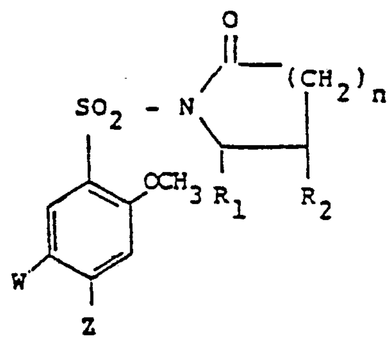

enthält, worin n, $R_1$ und $R_2$ die in Anspruch 1 angegebenen Bedeutungen hat und Z H oder $OCH_3$ darstellt, und W H, Cl oder $OCH_3$ darstellt.

17. Zusammensetzung nach einem der Ansprüche 12 oder 13, dadurch gekennzeichnet, daß sie eine Verbindung der allgemeinen Formel

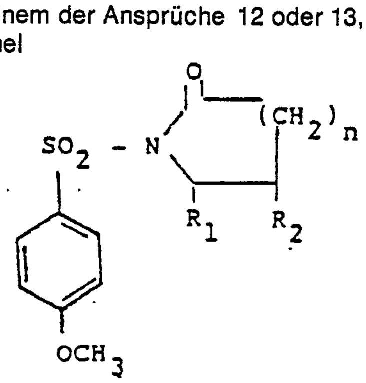

enthält, worin n, $R_1$ und $R_2$ die in den Ansprüchen 1 bis 3 angegebenen Bedeutungen haben.

18. Zusammensetzung nach einem der Ansprüche 12 oder 13, dadurch gekennzeichnet, daß sie eine Verbindung der allgemeinen Formel

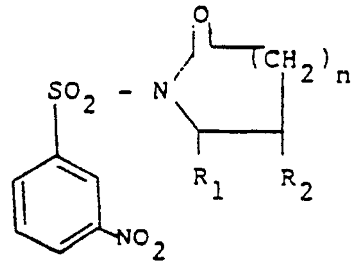

enthält, worin n, $R_1$ und $R_2$ die in den Ansprüchen 1 bis 3 angegebenen Bedeutungen haben.

19. Pharmazeutische Zusammensetzung nach einem der Ansprüche 12 bis 18, mit psychotroper Wirkung, dadurch gekennzeichnet, daß sie in zu verabreichenden Tagesdosen von 100 bis 1 000 mg aktiver Substanz verpackt ist.

20. Pharmazeutische Zusammensetzung nach einem der Ansprüche 12 bis 18, dadurch gekennzeichnet, daß sie in einheitlicher Darreichungsform einer Kapsel oder Tablette, mit 25 bis 200 mg aktiver Substanz, vorliegt.

21. Pharmazeutische Zusammensetzung nach einem der Ansprüche 12 bis 18, dadurch gekennzeichnet, daß sie in einheitlicher Darreichungsform einer trinkbaren Lösung, geeignet für die einheitliche Dosierung von 25 bis 200 mg aktiver Substanz, vorliegt.